# EUROPEAN PATENT APPLICATION

(11) **EP 3 988 124 A1**
(43) Date of publication of application: **27.04.2022**
(21) Application number: 20940475.5
(22) Date of filing: 27.08.2020
(51) Int. Cl.: A61K 49/22, A61K 9/00, A61K 41/00, A61K 47/54, A61K 47/69, A61K 47/62, A61K 47/61, A61K 47/60

(54) **ULTRASOUND-GUIDED DRUG DELIVERY SYSTEM USING ULTRASOUND CONTRAST MEDIUM CONTAINING LIGAND HAVING DRUG IMMOBILIZED THERETO THROUGH ESTER BOND**

(71) Applicant: Sahmyook University Industry-Academic Cooperation Foundation, Seoul 01795 (KR); N to B Co., Ltd., Seoul 01795 (KR)
(72) Inventor: PARK, Myoung Hwan, Namyangju-si Gyeonggi-do 12110 (KR)
(74) Representative: dompatent von Kreisler Selting Werner - Partnerschaft von Patent- und Rechtsanwälten mbB
(86) International application number: PCT/KR2020/011512
(87) International publication number: WO 2022/045398

(57) **Abstract**

Proposed are an ultrasound-assisted drug delivery carrier containing a ligand linked with a drug through an ester bond, a phospholipid, and a PEGylated phospholipid, a composition for drug delivery including the drug delivery carrier, and a method of preventing or treating a disease including administering the composition to an individual other than a human and releasing a drug by subjecting the site of administration of the composition to ultrasound irradiation. The ultrasound-assisted drug delivery carrier containing a ligand linked with a drug through an ester bond, a phospholipid, and a PEGylated phospholipid can be provided in the form of microbubbles or nanobubbles, and is capable of accelerating drug release due to collapse of the bubbles and promotion of hydrolysis of the ester bond during ultrasound irradiation, making it possible to deliver the drug to a desired site with high efficiency.

## Description

### Technical Field

The present disclosure provides an ultrasound-assisted drug delivery carrier containing a ligand linked with a drug through an ester bond, a phospholipid, and a PEGylated phospholipid, a composition for drug delivery including the drug delivery carrier, and a method of preventing or treating a disease including administering the composition to an individual other than a human and releasing a drug by subjecting the site of administration of the composition to ultrasound irradiation.

### Background Art

A drug delivery system is aimed at maximizing the treatment efficacy and effect by selectively delivering a drug to a target site and optimizing the effective blood concentration for a long time in consideration of the type of disease, and at minimizing drug side effects. In order to increase the target delivery efficiency of such a drug delivery system, studies into maximizing drug delivery, such as linking various target ligands, controlling the drug release rate through near-infrared irradiation, improving the permeation ability using ultrasound, and the like, have been conducted in various fields, but the development of superior drug delivery technology to control the release of an appropriate amount of drug to the target site is still required.

Accordingly, the inventors of the present disclosure have made efforts to develop a drug delivery system capable of maximizing the effect of a drug by releasing an appropriate amount of the drug with high efficiency, and have ascertained that an ultrasound-assisted drug delivery carrier containing a ligand (a phospholipid, a biomaterial, or an amphipathic material) linked with a drug through an ester bond, a phospholipid, and a PEGylated phospholipid is provided in the form of microbubbles or nanobubbles, and is capable of accelerating drug release due to collapse of the bubbles and promotion of hydrolysis of the ester bond during ultrasound irradiation, thus culminating in the present disclosure.

### Disclosure

### Technical Problem

Accordingly, the present disclosure is intended to solve the problems encountered in the related art and other problems associated therewith.

An exemplary objective of the present disclosure is to provide an ultrasound-assisted drug delivery carrier containing a ligand linked with a drug through an ester bond, a phospholipid, and a PEGylated phospholipid.

Another exemplary objective of the present disclosure is to provide a composition for drug delivery including the drug delivery carrier described above.

Still another exemplary objective of the present disclosure is to provide a method of preventing or treating a disease including administering the composition described above to an individual other than a human and releasing a drug by subjecting the site of administration of the composition to ultrasound irradiation.

The technical problem to be achieved according to the technical idea disclosed in the present specification is not limited to the objectives of solving the problems mentioned above, and other objectives not mentioned will be clearly apparent to a person skilled in the art from the following description.

### Technical Solution

Hereinafter, a detailed description will be given of the present disclosure. Meanwhile, descriptions and embodiments disclosed in the present application may be applied to other descriptions and embodiments. Specifically, all combinations of the various elements disclosed in the present application fall within the scope of the present application. In addition, it cannot be considered that the scope of the present application is limited by the following specific description.

In order to accomplish the above objectives, an aspect of the present disclosure provides an ultrasound-assisted drug delivery carrier containing a ligand linked with a drug through an ester bond, a phospholipid, and a PEGylated phospholipid. The ligand may be a phospholipid, a biomaterial, or an amphipathic material, and in the present disclosure, the ligand linked with the drug through an ester bond may be selectively attached at the interface of the microbubbles or nanobubbles together with the phospholipid after preparation.

In the present disclosure, the term "biomaterial" includes all materials, surfaces, and structures that interact with organ systems, and the biomaterial of the present disclosure includes a chemical structure capable of being linked with a drug or a fluorescent dye through an ester bond, for example, a hydroxyl group or a carboxyl group, suitable for forming a drug delivery carrier in the form of microbubbles or nanobubbles. Specifically, the biomaterial may be a protein or a biocompatible polymer. For example, the protein may be gelatin, collagen, fibrin, gluten, elastin, or albumin (bovine serum albumin (BSA), human serum albumin (HSA)), and more specifically albumin, but is not particularly limited thereto. In addition, the biocompatible polymer may be alginic acid, pectin, chitin, carrageenin, gellan gum, carboxymethyl cellulose, dextran, poly(caprolactone) (PCL), poly(lactic acid) (PLA), poly(glycolic acid) (PGA), poly(vinyl alcohol) (PVA), poly(acrylic acid) (PAA), or hyaluronic acid, and more specifically hyaluronic acid, but is not particularly limited thereto.

In the present disclosure, in particular, the drug linked to hyaluronic acid through an ester bond is hydrolyzed at a very slow reaction rate under physiological conditions (a pH of 7.1 to 7.4), so the effect of the released drug cannot be exhibited. However, when the site to which the drug delivery carrier is administered is subjected to ultrasound irradiation, the hydrolysis reaction may be accelerated due to physical stimulation and elevated high temperature/high pressure, and the drug release rate may be increased 10-100 times or more, so the drug may be sufficiently released to the extent of exhibiting a significant effect on the desired site.

In the present disclosure, the term "amphipathic material" refers to a material or compound configured to include both a hydrophilic portion and a hydrophobic portion therein, and may be specifically configured such that alkyl as the hydrophobic portion and PEG (polyethylene glycol) as the hydrophilic portion are linked through an ester bond. More specifically, the hydrophobic alkyl may be a chain-like alkyl group having 5 to 30 carbon atoms, and the PEG may have a chemical structure of 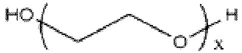 (in which x = 6 to 50). Moreover, the amphipathic material of the present disclosure has a chemical structure capable of being linked with a drug or a fluorescent dye through an ester bond, for example, a hydroxyl group or a carboxyl group, suitable for forming a drug delivery carrier in the form of microbubbles or nanobubbles.

In the present disclosure, in particular, the drug linked to the amphipathic material through an ester bond is hydrolyzed at a very slow reaction rate under physiological conditions (a pH of 7.1 to 7.4), so the effect of the released drug cannot be exhibited, but when the site to which the drug delivery carrier is administered is subjected to ultrasound irradiation, the hydrolysis reaction may be accelerated due to physical stimulation and elevated high temperature/high pressure, and the drug release rate may be increased 10-100 times or more, whereby the drug may be sufficiently released to the extent of exhibiting a significant effect on the desired site.

In the present disclosure, the term "phospholipid" refers to a kind of lipid containing phosphorus as a main component of a biomembrane, and has a structure in which two fatty acids and one phosphoric acid group are attached to glycerol. The type thereof varies depending on the type of organic material that is attached to the phosphoric acid group, and the side thereof to which the fatty acid is attached shows hydrophobicity and the side thereof to which the phosphoric acid group is attached shows hydrophilicity.

Specifically, in the present disclosure, the phospholipid may be at least one selected from the group consisting of dipalmitoylphosphatidylcholine (DPPC), dioleoylphosphatidylcholine (DOPC), distearoylphosphatidylcholine (DSPC), dimyristoylphosphatidylcholine (DMPC), didecanoylphosphatidylcholine (DDPC), dilauroylphosphatidylcholine (DLPC), dimyristoyl phosphatidylethanolamine (DMPE), dipalmitoyl phosphatidylethanolamine (DPPE), distearoyl phosphatidylethanolamine (DSPE), dioleyl phosphatidylethanolamine (DOPE), diarachidoyl phosphatidylethanolamine (DAPE), dilinoleyl phosphatidylethanolamine (DLPE), dipalmitoylphosphatidylglycerol (DPPG), dilauroyl phosphatidylglycerol (DLPG), distearoyl phosphatidylglycerol (DSPG), dioleoyl phosphatidylglycerol (DOPG), phosphatidylcholine (PC), and egg phosphatidylcholine (EPC), and more specifically dipalmitoylphosphatidylcholine (DPPC) or distearoyl phosphatidylethanolamine (DSPE), but is not particularly limited thereto.

In the present disclosure, in particular, the drug linked to the phospholipid through an ester bond is hydrolyzed at a very slow reaction rate under physiological conditions (a pH of 7.1 to 7.4), so the effect of the released drug cannot be exhibited. However, when the site to which the drug delivery carrier is administered is subjected to ultrasound irradiation, the hydrolysis reaction may be accelerated due to physical stimulation and elevated high temperature/high pressure, and the drug release rate may be increased 10-100 times or more, whereby the drug may be sufficiently released to the extent of exhibiting a significant effect on the desired site.

In the present disclosure, the term "ultrasound" refers to a sound wave with a frequency higher than 20 kHz, and for the purpose of the present disclosure, it is not particularly limited, but may be, for example, focused ultrasound, so long as a drug delivery carrier in the form of microbubbles or nanobubbles is irradiated therewith to thus cause collapse of the bubbles and generate physical stimulation and high temperature/high pressure so as to make it easy to release the drug or fluorescent dye.

In addition, the drug delivery carrier of the present disclosure includes a ligand linked with a drug through an ester bond, a phospholipid, and a PEGylated phospholipid, and may be provided in the form of microbubbles or nanobubbles having a diameter of 0.2 to 10 *µ*m. When the drug delivery carrier is subjected to ultrasound irradiation, hydrolysis of the ligand-drug ester bond is promoted due to physical stimulation and high temperature/high pressure caused by collapse of the bubbles, thereby accelerating the release of the drug.

In the present disclosure, the term "drug" may include all compounds, protein medicines, peptide medicines, nucleic acid molecules for gene therapy, nanoparticles, active ingredients for functional cosmetics, or cosmetically useful active ingredients, and for the purpose of the present disclosure, it is not particularly limited, so long as it is capable of binding to the ligand through an ester bond. Specifically, in the present disclosure, the drug is capable of directly binding to a hydroxyl group or a carboxyl group of the ligand constituting the drug delivery carrier through an ester bond, or is capable of binding by introducing an appropriate linker compound in the art that enables ester bonding between the ligand and the drug by a person skilled in the art.

When the drug of the present disclosure is a hydrophobic drug, the drug may be carried inside the drug delivery carrier based on a difference in hydrophilic/hydrophobic properties depending on the direction of the phospholipid, and the drug delivery carrier of the present disclosure is capable of stably carrying and delivering most hydrophobic drugs.

More specifically, examples of the drug may include, but are not limited to, anticancer agents, antiinflammatory agents, analgesics, anti-arthritis agents, antispasmodics, antidepressants, antipsychotic drugs, tranquilizers, anxiolytics, drug antagonists, anti-Parkinson's-disease drugs, cholinergic agonists, antiangiogenic agents, immunosuppressants, antiviral agents, antibiotics, appetite suppressants, anticholinergics, antihistamines, antimigraine agents, hormonal agents, coronary vasodilators, cerebrovascular vasodilators, peripheral vasodilators, contraceptives, antithrombotic agents, diuretics, antihypertensive agents, cardiovascular drugs, cosmetic ingredients (e.g. anti-wrinkling agents, skin-aging inhibitors, and skin-whitening agents), and the like. For example, the drug may be, but is not limited to, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, Adriamycin, methotrexate, busulfan, chlorambucil, cyclophosphamide, melphalan, nitrogen mustard, nitrosourea, or camptothecin.

Also, in the drug delivery carrier, a targeting agent (a targeting material) may be additionally introduced to the surface of the ligand or the phospholipid, whereby the efficiency of drug release may be further increased upon ultrasound irradiation. In the present disclosure, the term "targeting agent" refers to a molecule specific for a target, and may be an antibody or antibody fragment specific for a specific target, or an aptamer. The targeting agent is designed to specifically bind to targets including receptors, as well as organs, tissues, and cells. Therefore, the targeting agent may specifically bind to a receptor expressed in association with a specific disease, so the drug delivery carrier of the present disclosure is able to specifically bind to the target, and the drug linked through an ester bond may be released and delivered into the target.

In order to accomplish the above objectives, another aspect of the present disclosure provides a composition for drug delivery including the drug delivery carrier described above. Here, the biomaterial, amphipathic material, phospholipid, ultrasound, and drug are as described above.

In order to accomplish the above objectives, still another aspect of the present disclosure provides a method of preventing or treating a disease including administering the composition described above to an individual other than a human and releasing a drug by subjecting the site of administration of the composition to ultrasound irradiation.

In the present disclosure, the term "individual" refers to all animals including humans to which the drug delivery carrier or composition for drug delivery according to the present disclosure is capable of being administered for the prevention or treatment of diseases, and specifically all mammals including humans, but is not particularly limited thereto.

In the present disclosure, the term "administration" means introducing the drug delivery carrier or the composition for drug delivery to an individual in an appropriate manner, and the route of administration may be any general route, so long as it is able to reach the target site. Specifically, intraperitoneal administration, intravenous administration, intramuscular administration, subcutaneous administration, intradermal administration, oral administration, topical administration, intranasal administration, intrapulmonary administration, and intrarectal administration are possible, but the present disclosure is not particularly limited thereto.

In the present disclosure, the term "disease" may mean various brain diseases including brain tumors and all malignant tumors (cancers) in the human body, but is not particularly limited thereto. Specifically, the cancer may be pseudomyxoma peritonei, intrahepatic cholangiocarcinoma, hepatoblastoma, liver cancer, thyroid cancer, colon cancer, testicular cancer, myelodysplastic syndrome, glioblastoma, oral cancer, carcinoma of the lip, mycosis fungoides, acute myelogenous leukemia, acute lymphocytic leukemia, basal-cell carcinoma, ovarian epithelial carcinoma, ovarian germ cell carcinoma, male breast cancer, brain tumor, pituitary adenoma, multiple myeloma, gallbladder cancer, biliary tract cancer, colorectal cancer, chronic myelogenous leukemia, chronic lymphocytic leukemia, retinoblastoma, choroidal melanoma, diffuse giant B-cell lymphoma, cancer of ampulla of Vater, bladder cancer, peritoneal cancer, parathyroid cancer, adrenal cancer, paranasal sinus cancer, non-small cell lung cancer, non-Hodgkin's lymphoma, tongue cancer, astrocytoma, small-cell lung cancer, pediatric brain cancer, pediatric lymphoma, pediatric leukemia, small intestine cancer, meningioma, esophageal cancer, glioma, neuroblastoma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary site, gastric lymphoma, stomach cancer, gastric carcinoid tumor, gastrointestinal stromal tumor, Wilms' tumor, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoma, vaginal cancer, spinal carcinoma, vestibular schwannoma, pancreatic cancer, salivary gland cancer, Kaposi's sarcoma, Paget's disease, tonsillar cancer, squamous cell carcinoma, lung adenocarcinoma, lung cancer, squamous cell carcinoma of the lungs, skin cancer, anal cancer, rhabdomyosarcoma, laryngeal cancer, pleural cancer, or thymic cancer, but is not particularly limited thereto.

### Advantageous Effects

According to the present disclosure, an ultrasound-assisted drug delivery carrier containing a ligand linked with a drug through an ester bond, a phospholipid, and a PEGylated phospholipid can be provided in the form of microbubbles or nanobubbles, and is capable of accelerating drug release due to collapse of the bubbles and promotion of hydrolysis of the ester bond during ultrasound irradiation, making it possible to deliver the drug to a desired site with high efficiency.

However, the effects according to an embodiment of the technology disclosed in the present specification are not limited to those mentioned above, and other effects not mentioned herein will be clearly understood by those of ordinary skill in the art from the following description.

### Brief Description of Drawings

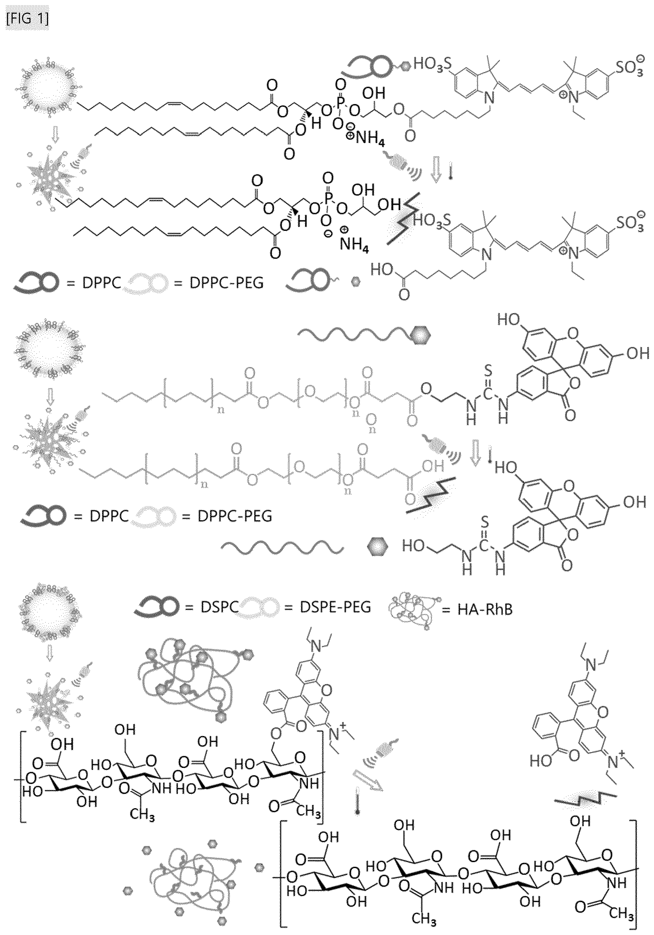

FIG. 1 schematically shows a process of accelerating drug release by promoting hydrolysis of an ester bond between a phospholipid and a drug due to the occurrence of high temperature and high pressure when each drug delivery carrier of the present disclosure is subjected to ultrasound irradiation;

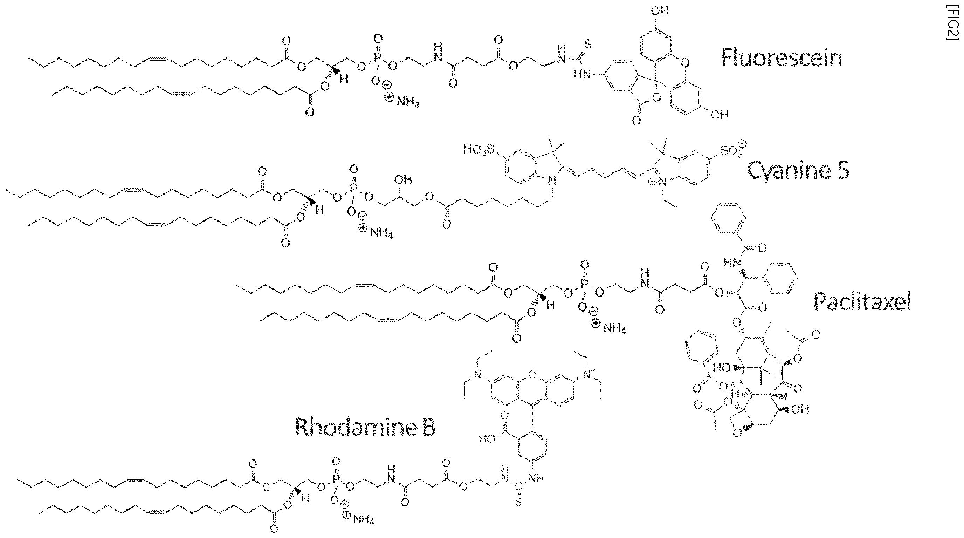

FIG. 2 shows a chemical structure in which a phospholipid and a drug (paclitaxel) or a model drug (fluorescein, cyanine 5, rhodamine B) are linked through an ester bond in the drug delivery carrier of the present disclosure;

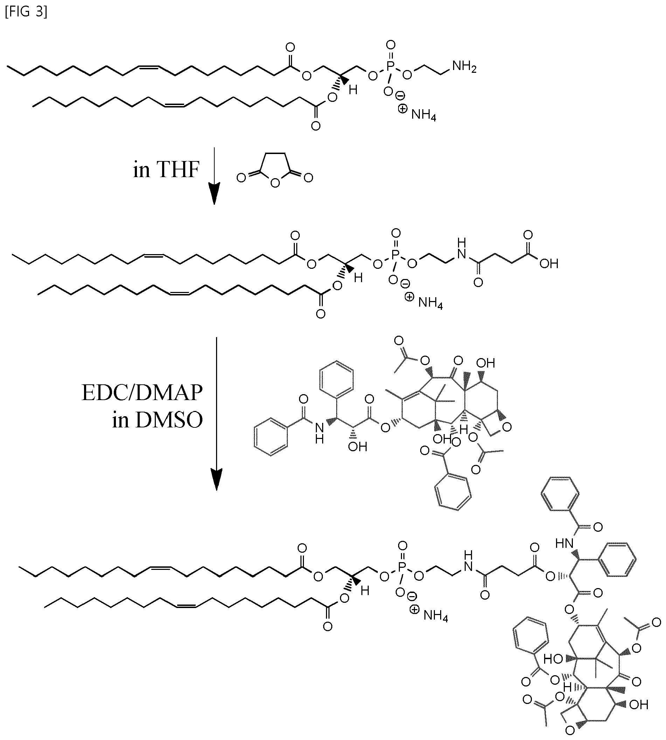

FIG. 3 schematically shows a process of synthesizing a phospholipid linked with a drug (paclitaxel) through an ester bond in the present disclosure;

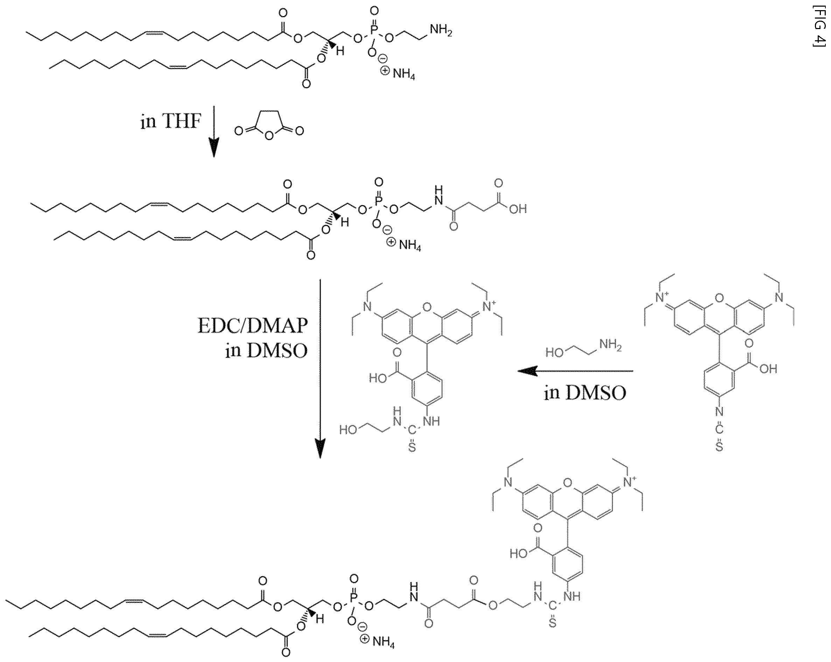

FIG. 4 schematically shows a process of synthesizing a phospholipid linked with a model drug (rhodamine B) through an ester bond in the present disclosure;

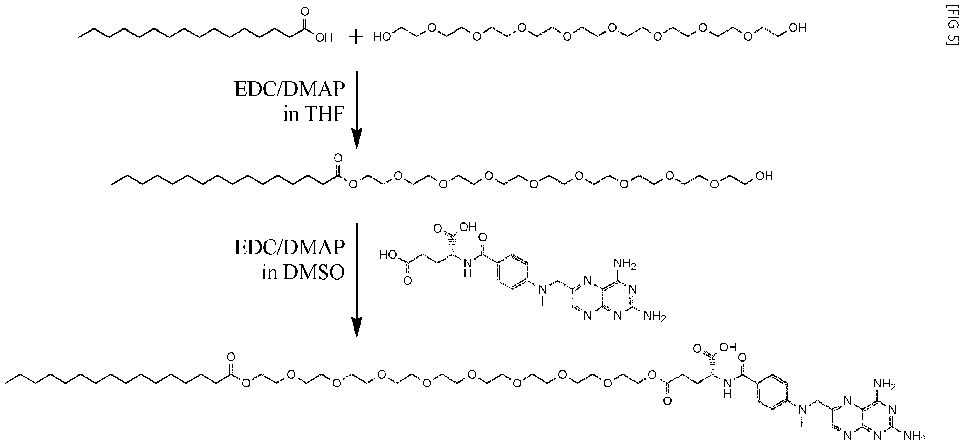

FIG. 5 schematically shows a process of synthesizing an amphipathic material (hydrophobic alkyl-PEG) linked with a drug (methotrexate) through an ester bond in the present disclosure;

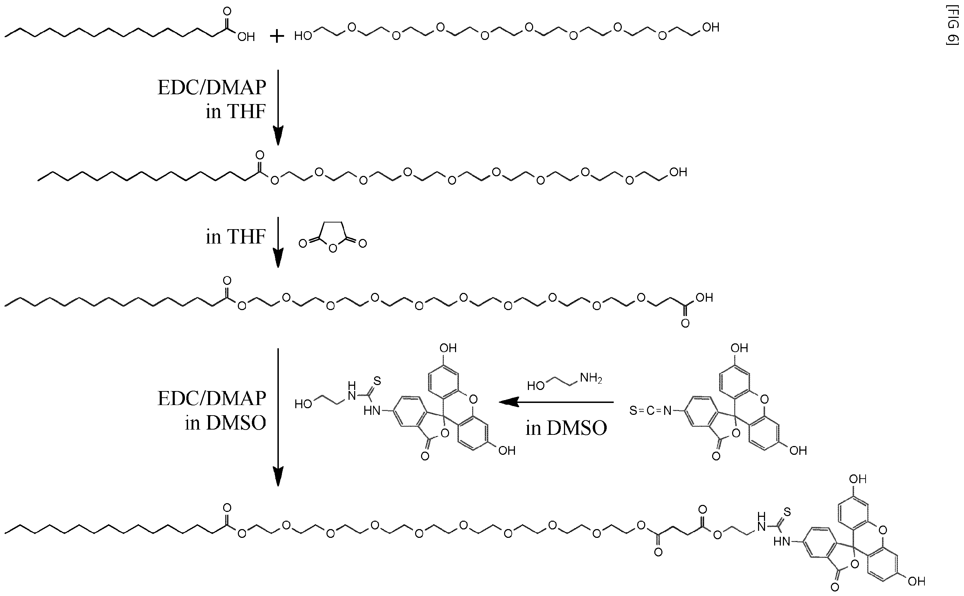

FIG. 6 schematically shows a process of synthesizing an amphipathic material (hydrophobic alkyl-PEG) linked with a model drug (fluorescein) through an ester bond in the present disclosure;

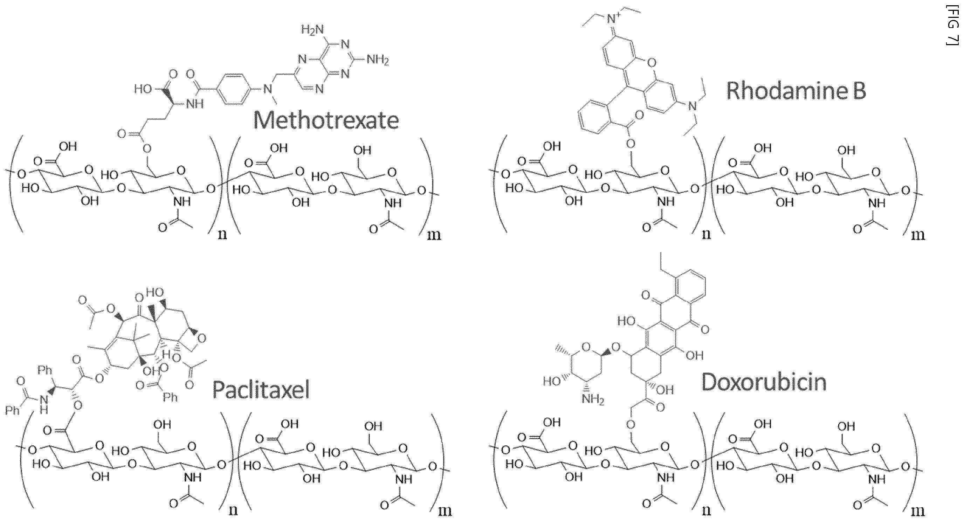

FIG. 7 shows a chemical structure in which a biomaterial (hyaluronic acid, HA) and a drug (methotrexate, paclitaxel, doxorubicin) or a model drug (rhodamine B) are linked through an ester bond in the drug delivery carrier of the present disclosure;

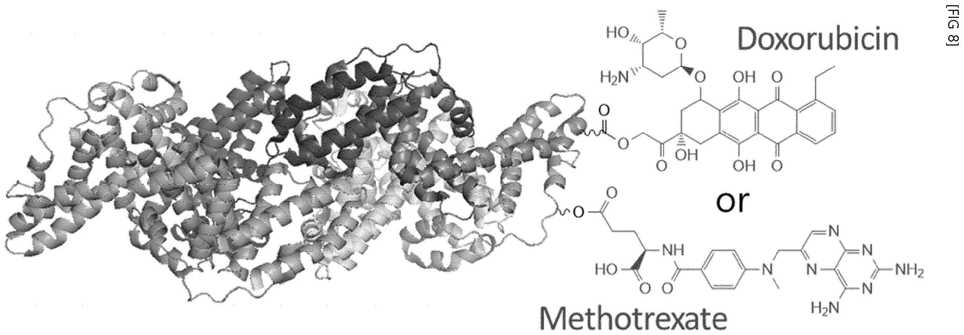

FIG. 8 shows the three-dimensional structure of a protein (albumin) and a structure in which a drug (doxorubicin or methotrexate) is linked through an ester bond in the drug delivery carrier of the present disclosure;

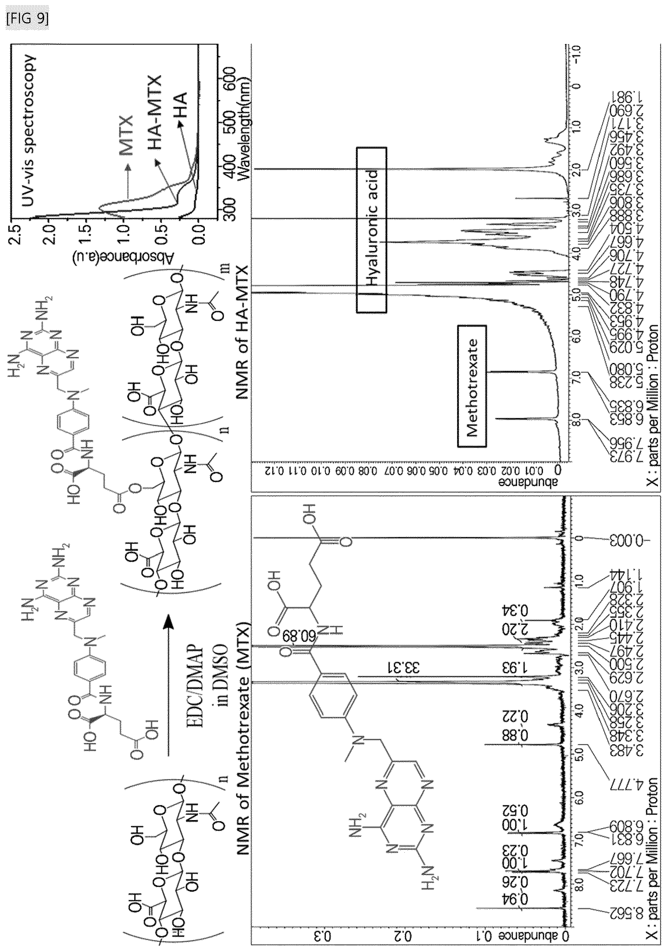

FIG. 9A schematically shows a process of synthesizing a biomaterial (hyaluronic acid, HA) linked with a drug (methotrexate) through an ester bond in the present disclosure, and FIG. 9B shows NMR and UV-vis analysis data of the biomaterial before and after synthesis;

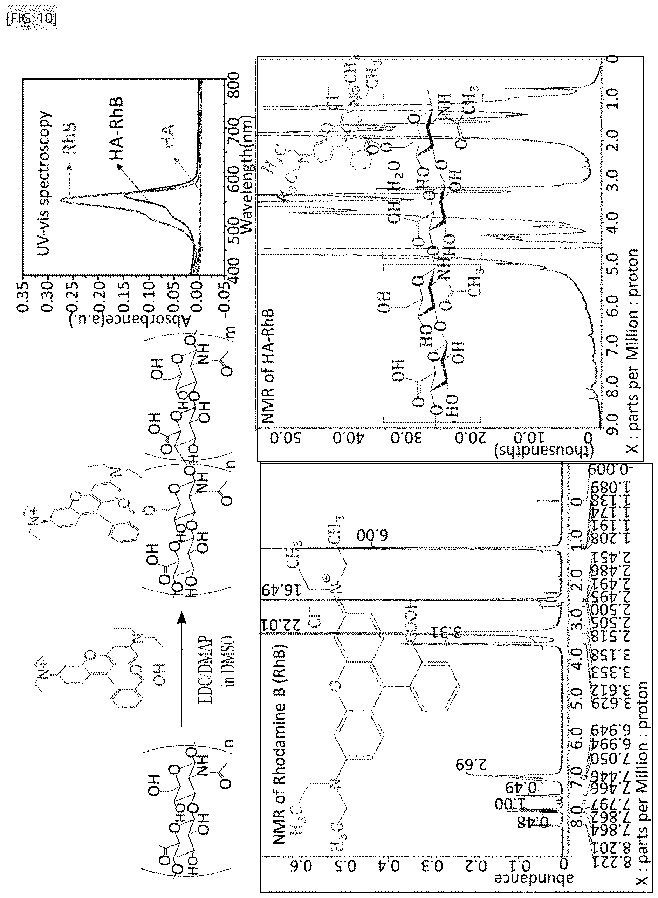

FIG. 10A schematically shows a process of synthesizing a biomaterial (hyaluronic acid, HA) linked with a model drug (rhodamine B) through an ester bond in the present disclosure, and FIG. 10B shows NMR and UV-vis analysis data of the biomaterial before and after synthesis;

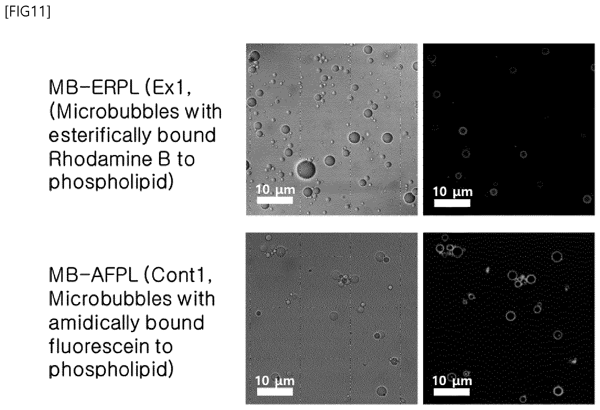

FIG. 11 shows optical microscope and confocal microscope images of a drug delivery carrier (an experimental group, MB-ERPL) containing a phospholipid linked with a model drug (rhodamine B) through an ester bond, and a drug delivery carrier (a control group, MB-AFPL) containing a phospholipid linked with a model drug (fluorescein) through an amide bond;

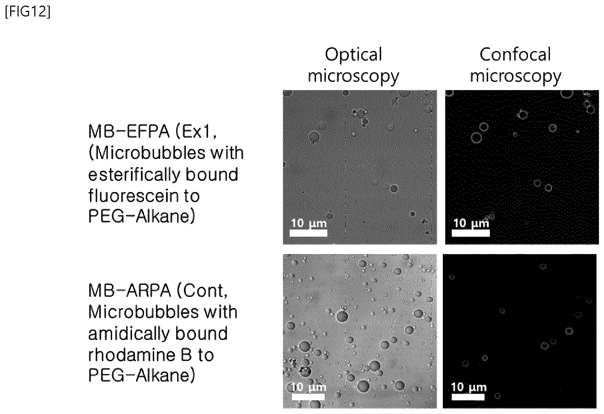

FIG. 12 shows optical microscope and confocal microscope images of a drug delivery carrier (an experimental group, MB-EFPA) containing an amphipathic material (hydrophobic alkyl-PEG) linked with a model drug (fluorescein) through an ester bond, and a drug delivery carrier (a control group, MB-ARPA) containing an amphipathic material (hydrophobic alkyl-PEG) linked with a model drug (rhodamine B) through an amide bond;

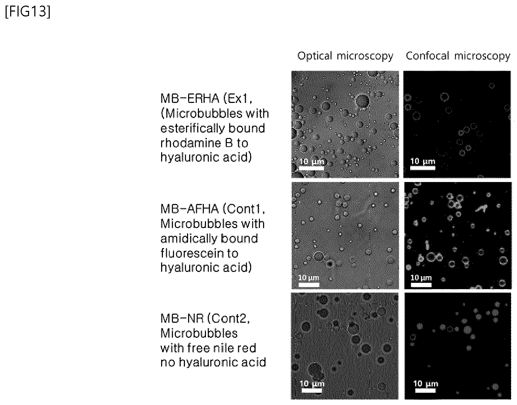

FIG. 13 shows optical microscope and confocal microscope images of a drug delivery carrier (an experimental group, MB-ERHA) containing a biomaterial (hyaluronic acid) linked with a model drug (rhodamine B) through an ester bond, and drug delivery carriers (control groups) containing a biomaterial linked with a model drug (fluorescein) through an amide bond and containing a model drug (Nile red) without hyaluronic acid;

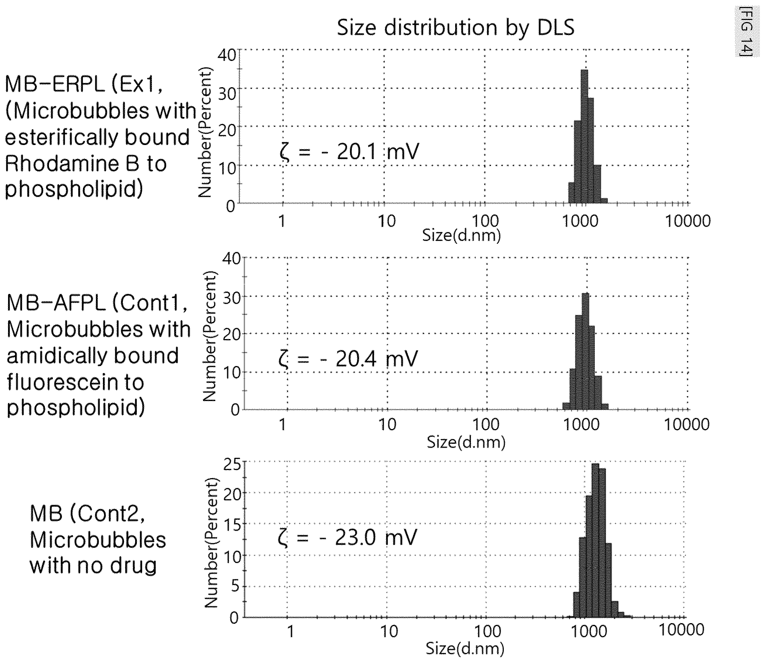

FIG. 14 shows the results of analysis of the size of the microbubble-based contrast agent using a DLS particle size analyzer, of a drug delivery carrier (an experimental group, MB-ERPL) containing a phospholipid linked with a model drug (rhodamine B) through an ester bond, a drug delivery carrier (Control Group 1, MB-AFPL) containing a phospholipid linked with a model drug (fluorescein) through an amide bond, and a drug delivery carrier (Control Group 2, MB) containing only a phospholipid and a PEGylated phospholipid;

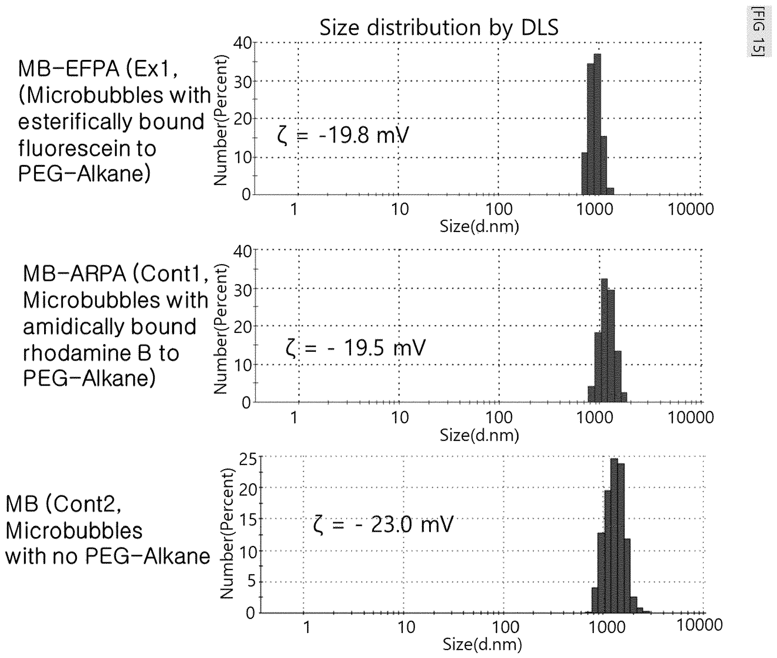

FIG. 15 shows the results of analysis of the size of the microbubble-based contrast agent using a DLS particle size analyzer, of a drug delivery carrier (an experimental group, MB-EFPA) containing an amphipathic material (hydrophobic alkyl-PEG) linked with a model drug (fluorescein) through an ester bond, a drug delivery carrier (Control Group 1, MB-ARPA) containing an amphipathic material (hydrophobic alkyl-PEG) linked with a model drug (rhodamine B) through an amide bond, and a drug delivery carrier (Control Group 2, MB) containing only a phospholipid and a PEGylated phospholipid;

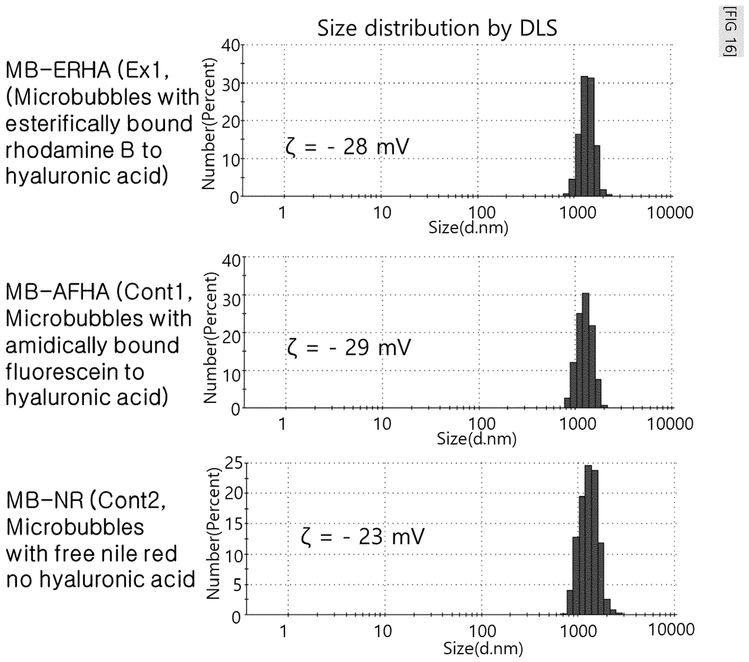

FIG. 16 shows the results of analysis of the size of the microbubble-based contrast agent using a DLS particle size analyzer, of a drug delivery carrier (an experimental group, MB-ERHA) containing a biomaterial (hyaluronic acid) linked with a model drug (rhodamine B) through an ester bond, a drug delivery carrier (Control Group 1, MB-AFHA) containing a biomaterial linked with a model drug (fluorescein) through an amide bond, and a drug delivery carrier (Control Group 2, MB-NR) containing a model drug (Nile red) without hyaluronic acid;

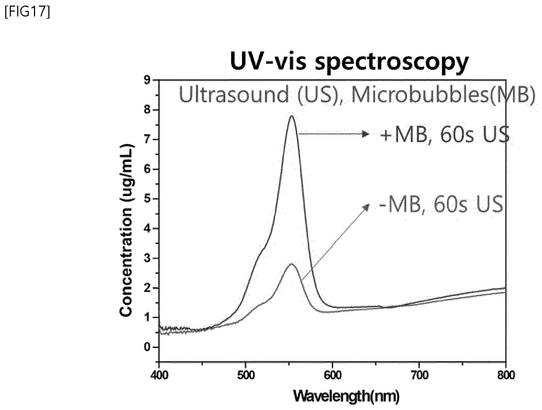

FIG. 17 shows the results of comparison of the amount of the model drug that is released through UV-vis analysis when a solution in which only a phospholipid (a control group, ERPL) linked with a model drug (rhodamine B) through an ester bond is dissolved and a drug delivery carrier (an experimental group, MB-ERPL) in the form of microbubbles containing a phospholipid linked with a model drug (rhodamine B) through an ester bond are subjected to ultrasound irradiation;

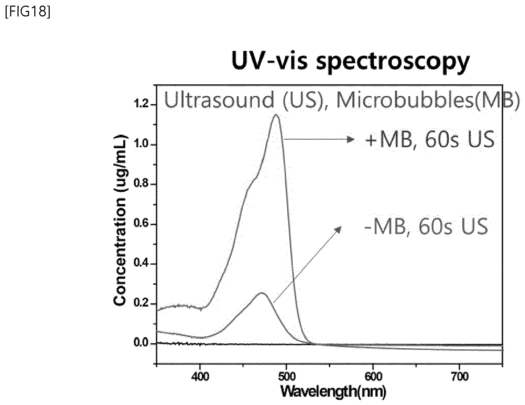

FIG. 18 shows the results of comparison of the amount of the model drug that is released through UV-vis analysis when a solution in which only an amphipathic material (a control group, EFPA) linked with a model drug (fluorescein) through an ester bond is dissolved and a drug delivery carrier (an experimental group, MB-EFPA) in the form of microbubbles containing an amphipathic material linked with a model drug (fluorescein) through an ester bond are subjected to ultrasound irradiation;

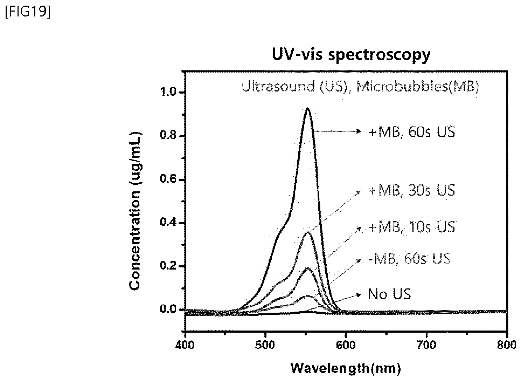

FIG. 19 shows the results of comparison of the amount of the model drug that is released through UV-vis analysis when a solution in which only a biomaterial (a control group, ERHA) linked with a model drug (rhodamine B) through an ester bond is dissolved and a drug delivery carrier (an experimental group, MB-ERHA) in the form of microbubbles containing a biomaterial linked with a model drug (rhodamine B) through an ester bond are subjected to ultrasound irradiation;

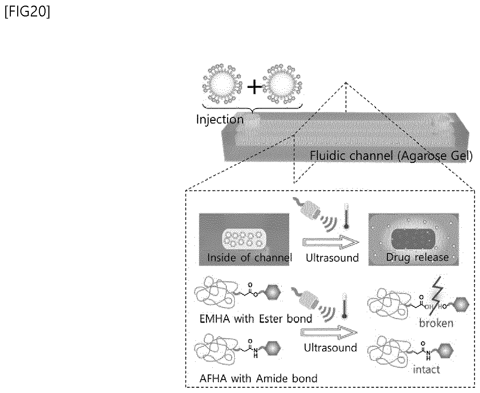

FIG. 20 schematically shows an experiment on the extent of diffusion of the drug into agarose gel during ultrasound irradiation for a drug delivery carrier (an experimental group) containing a ligand (a phospholipid, an amphipathic material, or a biomaterial) linked with a drug through an ester bond and a drug delivery carrier (a control group) containing a ligand linked with a drug through an amide bond;

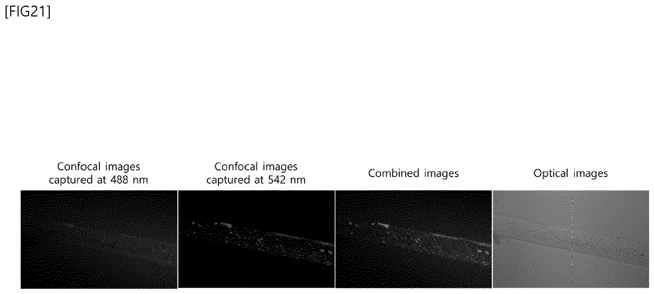

FIG. 21 shows the results of confocal microscopy to determine the extent of diffusion of the drug during ultrasound irradiation for a drug delivery carrier (an experimental group, MB-EFPA) containing an amphipathic material (hydrophobic alkyl-PEG) linked with a drug through an ester bond and a drug delivery carrier (a control group, MB-EMPA) containing an amphipathic material (hydrophobic alkyl-PEG) linked with a drug through an amide bond;

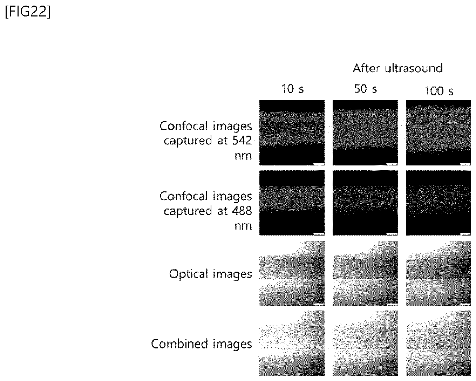

FIG. 22 shows the results of confocal microscopy to determine the extent of diffusion of the drug during ultrasound irradiation for a drug delivery carrier (an experimental group) containing a biomaterial (hyaluronic acid) linked with a drug through an ester bond and a drug delivery carrier (a control group) containing a biomaterial linked with a drug through an amide bond;

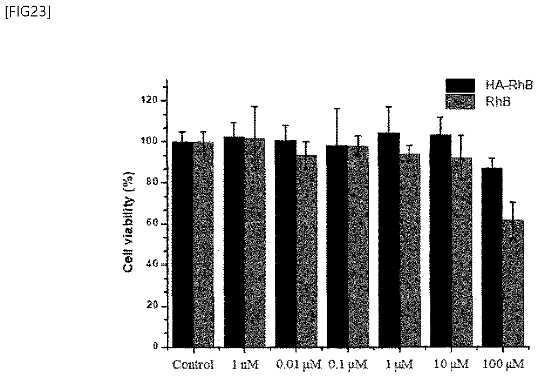

FIG. 23 shows the results of testing on *in-vitro* biotoxicity of a biomaterial linked with a model drug (rhodamine B) through an ester bond and a model drug;

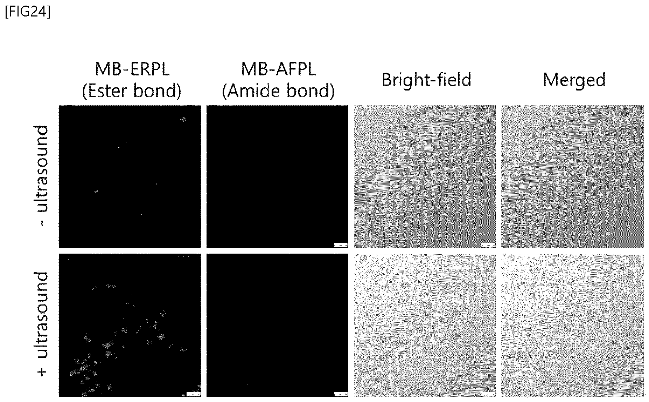

FIG. 24 shows the results of comparison of *in-vitro* cell absorption depending on whether or not ultrasound irradiation is performed on a drug delivery carrier (an experimental group, MB-ERPL) containing a phospholipid linked with a model drug (rhodamine B) through an ester bond and a drug delivery carrier (a control group, MB-AFPL) containing a phospholipid linked with a model drug (fluorescein) through an amide bond; and

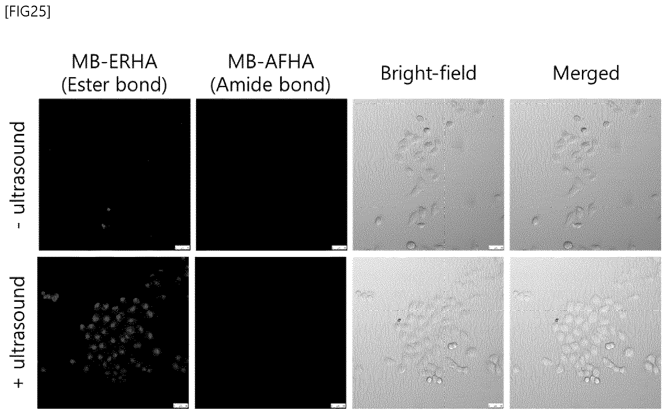

FIG. 25 shows the results of comparison of the *in-vitro* cell absorption depending on whether or not ultrasound irradiation is performed on a drug delivery carrier (an experimental group, MB-ERHA) containing a biomaterial linked with a model drug (rhodamine B) through an ester bond and a drug delivery carrier (a control group, MB-AFHA) containing a biomaterial linked with a model drug (fluorescein) through an amide bond.

### Mode for Disclosure

A better understanding of the present disclosure may be given through the following examples. However, these examples are merely set forth to illustrate the present disclosure, and are not to be construed as limiting the scope of the present disclosure.

### Example 1: Manufacture of microbubble drug delivery carrier

### 1-1: Manufacture of drug delivery carrier containing phospholipid linked with model drug through ester bond

A mixture of 1-10 mg of a PEG-conjugated phospholipid DSPE-PEG (2000) and 5-30 mg of a phospholipid (DSPC) at an appropriate ratio and 1-10 mg of a phospholipid linked with a drug through an ester bond were dispersed and dissolved in 2-5 mL of chloroform contained in a 30-mL vial. The vial containing the mixed solution was treated using a rotary concentrator to remove the organic solvent therefrom, thus forming a vial coated with an opaque phospholipid film. 3-5 mL of a sodium chloride (0.9%) aqueous solution and 1 mL of a liquefied gas (2H,3H-decafluoropentane) were added to the film-coated vial and ultrasound was applied thereto for 0.5-2 minutes using a tip sonicator, thereby manufacturing a microbubble drug delivery carrier. After the prepared aqueous solution was gradually cooled, the solution in the upper layer in which the residue remained was removed, and the precipitated microbubble drug delivery carrier was recovered. The microbubble solution thus prepared was confirmed through NTA, DLS, confocal microscopy, and SEM.

As a result, a drug delivery carrier (an experimental group, MB-ERPL) containing a phospholipid linked with a model drug (rhodamine B) through an ester bond was provided in the form of microbubbles having a diameter of 1-3 *µ*m, and the surface charge thereof exhibited a negative value (-20.1 mV) due to the presence of DSPE-PEG (2000), having a negative charge.

In addition, a drug delivery carrier (a control group, MB-AFPL) containing a phospholipid linked with a model drug (fluorescein) through an amide bond was provided in the form of microbubbles having a diameter of 1-3 *µ*m, and likewise, the surface charge thereof was about -20.4 mV due to the presence of DSPE-PEG (2000), having a negative charge.

Meanwhile, in the case of microbubbles (MB) containing only a phospholipid and a PEGylated phospholipid, microbubbles having a diameter of 1-5 *µ*m were formed, and the surface charge thereof was relatively small, particularly about -23 mV.

### 1-2: Manufacture of drug delivery carrier containing amphipathic material (hydrophobic alkyl-PEG) linked with model drug through ester bond

A mixture of 1-10 mg of a PEG-conjugated phospholipid DSPE-PEG (2000) and 5-30 mg of a phospholipid (DSPC) at an appropriate ratio and 1-10 mg of an amphipathic material (hydrophobic alkyl-PEG) linked with a drug through an ester bond were dispersed and dissolved in 2-5 mL of chloroform contained in a 30-mL vial. The vial containing the mixed solution was treated using a rotary concentrator to remove the organic solvent therefrom, thus forming a vial coated with an opaque phospholipid film. 3-5 mL of a sodium chloride (0.9%) aqueous solution and 1 mL of a liquefied gas (2H,3H-decafluoropentane) were added to the film-coated vial, and ultrasound was applied thereto for 0.5-2 minutes using a tip sonicator, thereby manufacturing a microbubble drug delivery carrier. After the prepared aqueous solution was gradually cooled, the solution in the upper layer in which the residue remained was removed, and the precipitated microbubble drug delivery carrier was recovered. The microbubble solution thus prepared was confirmed through NTA, DLS, confocal microscopy, and SEM.

As a result, a drug delivery carrier (an experimental group, MB-EFPA) containing an amphipathic material (hydrophobic alkyl-PEG) linked with a model drug (fluorescein) through an ester bond was provided in the form of microbubbles having a diameter of 1-3 *µ*m, and the amphipathic material (green) was selectively formed on the surface of the bubbles. The surface charge thereof exhibited a negative value (-19.8 mV) due to the presence of DSPE-PEG (2000), having a negative charge.

In addition, a drug delivery carrier (a control, MB-ARPA) containing an amphipathic material (hydrophobic alkyl-PEG) linked with a model drug (rhodamine B) through an amide bond was provided in the form of microbubbles having a diameter of 1-3 *µ*m, and the amphipathic material (red) was selectively formed on the surface of the bubbles. Likewise, the surface charge thereof was about -19.5 mV due to the presence of DSPE-PEG (2000), having a negative charge.

Meanwhile, in the case of microbubbles (MB) containing only a phospholipid and a PEGylated phospholipid, microbubbles having a diameter of 1-5 *µ*m were formed, and the surface charge thereof was relatively small, particularly about -23 mV.

### 1-3: Manufacture of drug delivery carrier containing biomaterial (hyaluronic acid) linked with model drug through ester bond

A mixture of 1-10 mg of a PEG-conjugated phospholipid DSPE-PEG (2000) and 5-30 mg of a phospholipid (DSPC) at an appropriate ratio was dispersed and dissolved in 2-5 mL of chloroform contained in a 30-mL vial. 1-10 µg of a biomaterial (hyaluronic acid, HA) linked with a drug through an ester bond was dispersed and dissolved in 1-3 mL of a 40% ethanol aqueous solution, after which the resulting solution was mixed with the phospholipid solution prepared above. The vial containing the mixed solution was treated using a rotary concentrator to remove the organic solvent and water therefrom, thus forming a vial coated with an opaque phospholipid film. 3-5 mL of a sodium chloride (0.9%) aqueous solution and 1 mL of a liquefied gas (2H,3H-decafluoropentane) were added to the film-coated vial and ultrasound was applied thereto for 0.5-2 minutes using a tip sonicator, thereby manufacturing a microbubble drug delivery carrier. After the prepared aqueous solution was gradually cooled, the solution in the upper layer in which the residue remained was removed, and the precipitated microbubble drug delivery carrier was recovered. The microbubble solution thus prepared was confirmed through NTA, DLS, confocal microscopy, and SEM.

As a result, a drug delivery carrier (an experimental group, MB-ERHA) containing a biomaterial (hyaluronic acid) linked with a model drug (rhodamine B) through an ester bond was provided in the form of microbubbles having a diameter of 1-3 *µ*m, and hyaluronic acid having a negative charge (HA-RhB, red) was selectively formed on the surface of the bubbles, and the surface charge thereof showed a negative value (-28 mV) due to the presence of DSPE-PEG (2000) and hyaluronic acid, having a negative charge.

In addition, a drug delivery carrier (a control, MB-AFHA) containing a biomaterial linked with a model drug (fluorescein) through an amide bond was provided in the form of microbubbles having a diameter of 1-3 *µ*m, and hyaluronic acid having a negative charge (HA-fluoresceine, green) was selectively formed on the surface of the bubbles, and likewise, the surface charge thereof was about -29 mV due to the presence of DSPE-PEG (2000) and hyaluronic acid, having a negative charge.

Meanwhile, in the case of microbubbles containing a fluorescent dye (Nile red) without hyaluronic acid, microbubbles having a diameter of 1-5 *µ*m were formed, and the uniformity of the bubbles was decreased due to the presence of hydrophobic Nile red. Moreover, the bubbles in the form of a ring were mostly observed to have a spherical shape because Nile red was present only on the surface of the bubbles, but Nile red was capable of being dissolved in liquefied gas. Since DSPE-PEG was negatively charged, the surface charge of the microbubbles had a negative value, but was relatively small, particularly about -23 mV, due to the absence of hyaluronic acid.

### Example 2: Manufacture of microbubble drug delivery carrier using contrast agent

1-10 mg of a phospholipid, an amphipathic material (hydrophobic alkyl-PEG) or a biomaterial (hyaluronic acid, HA) linked with a drug through an ester bond was dissolved in 5 mL of a sodium chloride (0.9%) aqueous solution. The sodium chloride solution in which the drug was dissolved was placed in a syringe, injected into a container containing a SonoVue powder via a rubber stopper, and then vigorously stirred for 20 seconds or more using a vortex mixer (SonoVue powder: sulfur hexafluoride, Macrogol 4000, distearoylphosphatidylcholine, dipalmitoylphosphatidylglycerol sodium, and palmitic acid).

### Example 3: Experiment on drug release according to ultrasound irradiation

The solution of an experimental group (a drug delivery carrier containing the phospholipid, amphipathic material or biomaterial linked with the red model drug through an ester bond) dispersed in PBS and the solution of a control group (the phospholipid, amphipathic material or biomaterial dispersed without microbubbles) dispersed in PBS were placed in respective dialysis membrane bags. Each of the dialysis membrane bags containing the materials was placed in a beaker containing a PBS solution, followed by ultrasound irradiation. In the experimental group, the concentration of the released drug in the beaker was analyzed after irradiation for 10, 30 or 60 seconds, and in the control group, the concentration of the released drug was analyzed after sufficient irradiation for 60 seconds.

### 3-1: Drug delivery carrier containing phospholipid linked with model drug through ester bond

As a result, in the control group without microbubbles, a small amount of drug was released through ultrasound irradiation, but in the experimental group, manufactured with microbubbles, it was confirmed that at least three times the amount of the drug was released compared to the control group (FIG. 17).

### 3-2: Drug delivery carrier containing amphipathic material (hydrophobic alkyl-PEG) linked with model drug through ester bond

In the control group without microbubbles, a small amount of drug was released through ultrasound irradiation, but in the experimental group, manufactured with microbubbles, it was confirmed that at least five times the amount of the drug was released compared to the control group (FIG. 18).

### 3-3: Drug delivery carrier containing biomaterial (hyaluronic acid) linked with model drug through ester bond

In the control group without microbubbles, a small amount of drug was released through ultrasound irradiation, but in the experimental group, manufactured with microbubbles, it was confirmed that at least ten times the amount of the drug was released compared to the control group, and this release rate was observed to depend on the ultrasound irradiation time (FIG. 19).

### Example 4: Drug diffusion experiment according to ultrasound irradiation

The solution of each of an experimental group (a drug delivery carrier containing an amphipathic material or biomaterial linked with a model drug through an ester bond) and a control group (a drug delivery carrier containing an amphipathic material or biomaterial linked with a model drug through an amide bond), dispersed in PBS, was injected into a channel formed of hydrophilic agarose gel. Thereafter, ultrasound was applied onto the channel into which the solution was injected to thereby break the microbubbles, and accordingly, the diffusion of each model drug into the hydrophilic agarose gel and the extent thereof were evaluated.

### 4-1: Drug delivery carrier containing amphipathic material linked with model drug through ester bond

As a result, the hydrolysis reaction of the ester bond in the drug delivery carrier was accelerated due to physical stimulation through ultrasound irradiation and the local high temperature/high pressure caused by collapse of the microbubbles, so it was confirmed with a confocal analyzer that the green model drug (fluorescein) that was linked in the experimental group was rapidly released, and the released model drug permeated and was dispersed into the hydrophilic agarose gel.

On the other hand, in the case of the red model drug (rhodamine B) conjugated through an amide bond in the control group, the amide bond was almost completely undecomposed even under high-temperature/high-pressure conditions through ultrasound irradiation, so the drug was not released but was attached to the amphipathic material (hydrophobic alkyl-PEG) and thus maintained as a macromolecule, indicating that the permeation thereof into the hydrophilic agarose gel was weak (FIG. 21).

### 4-2: Drug delivery carrier containing biomaterial linked with model drug through ester bond

The hydrolysis reaction of the ester bond in the drug delivery carrier was accelerated due to the physical stimulation through ultrasound irradiation and the local high temperature/high pressure caused by collapse of the microbubbles, so it was confirmed with a confocal analyzer that the red model drug (rhodamine B) that was linked in the experimental group was rapidly released, and the released model drug permeated and was dispersed into the hydrophilic agarose gel.

On the other hand, in the case of the green model drug (fluorescein) conjugated through an amide bond in the control group, the amide bond was almost completely undecomposed even under high-temperature/high-pressure conditions through ultrasound irradiation, so the drug was not released but was attached to the biomaterial (hyaluronic acid) and thus maintained as a macromolecule, indicating that the permeation thereof into the hydrophilic agarose gel was weak (FIG. 22).

### Example 5: In-vitro cell experiment

### 5-1: Cytotoxicity test for biomaterial linked with model drug (rhodamine B) through ester bond

The biomaterial (HA-RhB) linked with the model drug (rhodamine B) through an ester bond and the model drug were added to U-251 MG cells such that the final concentration was adjusted to 1 nM, 0.01 µM, 0.1 µM, 1 µM, 10 µM and 100 µM, after which the cells were cultured for 24 hours. Thereafter, in order to confirm the *in-vitro* cytotoxicity of each material, cell viability was measured by adding a solution including a tetrazolium salt derivative and a cell culture medium thereto. As a result, free rhodamine B exhibited almost no toxicity at a low concentration, but toxicity thereof was observed at a concentration of 1 µM or more. On the other hand, toxicity of the biomaterial (HA-RhB) linked with the model drug through the ester bond was observed from a very high concentration, particularly 100 µM. This shows superior biocompatibility of the manufactured drug delivery carrier due to the characteristics of the hyaluronic acid platform, having superior biocompatibility (FIG. 23).

### 5-2: Analysis of intracellular absorption according to ultrasound irradiation

The solution of each of the experimental group (the drug delivery carrier containing the phospholipid or biomaterial linked with the red model drug (rhodamine B) through an ester bond) and the control group (the drug delivery carrier containing the phospholipid or biomaterial linked with the green model drug (fluorescein) through an amide bond) was used to analyze the extent of *in-vitro* intracellular absorption depending on whether or not ultrasound irradiation was performed. The experimental group and the control group were added together to U-251 MG cells, and the cell culture medium was replaced with a fresh medium before observation in order to remove materials that were not absorbed to the cells, followed by observation with a confocal microscope.

### 5-2-1: Drug delivery carrier containing phospholipid linked with red model drug through ester bond

As a result, both the experimental group and the control group showed slow cell absorption before ultrasound irradiation, so almost none of red and green fluorescent colors appeared. However, it was confirmed with a confocal analyzer that the red model drug (rhodamine B) that was linked was rapidly released and absorbed by cells because of the accelerated hydrolysis reaction of the ester bond in the drug delivery carrier of the experimental group due to the physical stimulation through ultrasound irradiation and the local high temperature/high pressure caused by collapse of the microbubbles.

On the other hand, in the case of the green model drug (fluorescein) conjugated through an amide bond in the control group, it was confirmed with a confocal analyzer that the amide bond was almost completely undecomposed even under high-temperature/high-pressure conditions due to collapse of the bubbles through ultrasound irradiation, so the drug was not released but was attached to the phospholipid and thus maintained as a macromolecule, and thus cell absorption still proceeded slowly (FIG. 24).

### 5-2-2: Drug delivery carrier containing biomaterial linked with red model drug through ester bond

Both the experimental group and the control group showed slow cell absorption due to the high-molecular-weight hyaluronic acid microbubble platform before ultrasound irradiation, so almost none of red and green fluorescent colors appeared. However, it was confirmed with a confocal analyzer that the red model drug (rhodamine B) that was linked was rapidly released and absorbed by cells because of the accelerated hydrolysis reaction of the ester bond in the drug delivery carrier of the experimental group due to the physical stimulation through ultrasound irradiation and the local high temperature/high pressure caused by collapse of the microbubbles.

On the other hand, in the case of the green model drug (fluorescein) conjugated through an amide bond in the control group, it was confirmed with a confocal analyzer that the amide bond was almost completely undecomposed even under high-temperature/high-pressure conditions due to collapse of the bubbles through ultrasound irradiation, so the drug was not released but was attached to the biomaterial (hyaluronic acid) and thus maintained as a macromolecule, and thus cell absorption still proceeded slowly (FIG. 25).

## Claims

1. An ultrasound-assisted drug delivery carrier comprising a ligand linked with a drug through an ester bond, a phospholipid, and a PEGylated phospholipid.

2. The drug delivery carrier of claim 1, wherein the ligand is a phospholipid, a biomaterial, or an amphipathic material.

3. The drug delivery carrier of claim 2, wherein the biomaterial is a protein or a biocompatible polymer.

4. The drug delivery carrier of claim 3, wherein the protein is gelatin, collagen, fibrin, gluten, elastin, or albumin, and the biocompatible polymer is alginic acid, pectin, chitin, carrageenin, gellan gum, carboxymethyl cellulose, dextran, PCL, PLA, PGA, PVA, PAA, or hyaluronic acid.

5. The drug delivery carrier of claim 2, wherein the amphipathic material is configured such that a hydrophobic alkyl and PEG (polyethylene glycol) are linked through an ester bond.

6. The drug delivery carrier of claim 5, wherein the hydrophobic alkyl is a chain-like alkyl group having 5 to 30 carbon atoms, and the PEG is 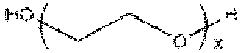 (wherein x = 6 to 50) .

7. The drug delivery carrier of claim 1, wherein the phospholipid is at least one selected from the group consisting of dipalmitoylphosphatidylcholine (DPPC), dioleoylphosphatidylcholine (DOPC), distearoylphosphatidylcholine (DSPC), dimyristoylphosphatidylcholine (DMPC), didecanoylphosphatidylcholine (DDPC), dilauroylphosphatidylcholine (DLPC), dimyristoyl phosphatidylethanolamine (DMPE), dipalmitoyl phosphatidylethanolamine (DPPE), distearoyl phosphatidylethanolamine (DSPE), dioleyl phosphatidylethanolamine (DOPE), diarachidoyl phosphatidylethanolamine (DAPE), dilinoleyl phosphatidylethanolamine (DLPE), dipalmitoylphosphatidylglycerol (DPPG), dilauroyl phosphatidylglycerol (DLPG), distearoyl phosphatidylglycerol (DSPG), dioleoyl phosphatidylglycerol (DOPG), phosphatidylcholine (PC), and egg phosphatidylcholine (EPC).

8. The drug delivery carrier of claim 1, wherein the drug delivery carrier is provided in a form of microbubbles or nanobubbles having a diameter of 0.2 to 10 *µ*m.

9. The drug delivery carrier of claim 1, wherein the drug delivery carrier accelerates release of the drug due to collapse of bubbles and promotion of hydrolysis of the ester bond through ultrasound irradiation.

10. The drug delivery carrier of claim 1, wherein a targeting agent (a targeting material) is additionally introduced on a surface of the ligand or the phospholipid.

11. A composition for drug delivery comprising the drug delivery carrier of any one of claims 1 to 10.

12. A method of preventing or treating a disease, comprising:
administering the composition for drug delivery of claim 11 to an individual other than a human; and
releasing a drug by subjecting a site of administration of the composition to ultrasound irradiation.
